# EUROPEAN PATENT APPLICATION

(11) **EP 4 397 646 A1**
(43) Date of publication of application: **10.07.2024**
(21) Application number: 22864015.7
(22) Date of filing: 22.06.2022
(51) Int. Cl.: C07C 1/12, C07C 9/04, C10L 3/08

(54) **SYSTEM AND METHOD FOR CONVERTING CO2 INTO FUEL**

(30) Priority: 01.09.2021 JP 2021142771
(71) Applicant: Hitachi, Ltd., Tokyo 100-8280 (JP)
(72) Inventor: SUGIMASA, Masatoshi, Tokyo 100-8280 (JP); GUNJI, Akira, Tokyo 100-8280 (JP)
(74) Representative: Mewburn Ellis LLP
(86) International application number: PCT/JP2022/024909
(87) International publication number: WO 2023/032427

(57) **Abstract**

A system of the present invention includes: a water electrolysis apparatus that generates hydrogen by electrolyzing water vapor using a fluctuating electric power derived from renewable energy; a reactor that reacts CO₂ with the hydrogen to generate hydrocarbon; an evaporator that evaporates water, by heat generated by the reaction in the reactor, to generate water vapor; a heat exchanger that heats the water vapor with exhaust gas from the water electrolysis apparatus and supplies the water vapor to the water electrolysis apparatus; and a heat storage unit for the reactor, and the heat storage unit including a heat storage material that stores heat generated by hydrocarbonization.

## Description

### Technical Field

The present invention relates to a system for reacting CO₂ with green hydrogen to convert the resultant into hydrocarbon.

### Background Art

As a technique for storing renewable energy while utilizing CO₂, a process is known in which water is electrolyzed by electric power derived from the renewable energy, and the obtained green hydrogen is reacted with CO₂ to convert CO₂ into methane. For example, JP 2018-537532 W discloses a method in which electric energy required for electrolysis is extracted from a renewable energy source, for example, wind energy, in which a catalyst used for methanation is disposed on a carrier structure having a high heat storage capacity, preferably formed as a honeycomb structure, and the carrier structure is used as a heat storage material for reaction heat generated during methanation. In addition, JP 2018-116834 A describes that a heat storage material is combined with hybrid use of steam electrolysis and steam fuel cell power generation to maintain a high temperature.

### Citation List

### Patent Literature

PTL 1: JP 2018-537532 W
PTL 2: JP 2018-116834 A

### Summary of Invention

### Technical Problem

The system for the methanation reaction described above supplies water to a reactor that reacts hydrogen with CO₂ to maintain a temperature suitable for the methanation reaction. Furthermore, the system supplies water vapor generated from water in the course of regulation of the temperature of the reactor to a high temperature steam electrolysis field utilizing a fluctuating electric power derived from renewable energy to generate hydrogen, and thus to supply the hydrogen to the reactor.

However, when the electric power decreases due to the fluctuating electric power, electrolysis of water vapor does not proceed, a hydrogen production amount is insufficient, and a methane production amount in the reactor decreases. Accordingly, there is a problem that an amount of water vapor also decreases and electrolysis of water vapor does not further proceed. This problem is not taken into consideration in the prior art described above. Thus, an object of the present invention is to provide a system and a method capable of stably producing a hydrocarbon with high efficiency from fluctuating renewable energy power by combining a hydrocarbonization reaction of CO₂ and water vapor electrolysis.

### Solution to Problem

In order to achieve the above object, the present invention is a system for converting CO₂ into fuel, the system including: a water electrolysis apparatus that generates hydrogen by electrolyzing water vapor using a fluctuating electric power derived from renewable energy; a reactor that reacts CO₂ with the hydrogen to generate hydrocarbon; an evaporator that evaporates water, by heat generated by the reaction in the reactor, to generate water vapor; a heat exchanger that heats the water vapor with exhaust gas from the water electrolysis apparatus and supplies the water vapor to the water electrolysis apparatus; and a heat storage unit for the reactor, the heat storage unit including a heat storage material that stores heat generated by the reaction.

In addition, the present invention is a method for converting CO₂ into fuel, the method including: an electrolysis step of electrolyzing water vapor using a fluctuating electric power derived from renewable energy to generate hydrogen; a hydrocarbonization step of reacting CO₂ with the hydrogen to generate hydrocarbon; a water vapor generation step of evaporating water, by heat generated by the reaction for generating the hydrocarbon, to generate water vapor; a water vapor heating step of heating the water vapor generated in the water vapor generation step with a high-temperature gas generated in the electrolysis step and then supplying the water vapor to the electrolysis step; and a heat storage step of storing the heat generated in the hydrocarbonization step in a heat storage material and radiating heat from the heat storage material to continue the hydrocarbon step and the water vapor generation step when the fluctuating electric power decreases.

### Advantageous Effects of Invention

According to the present invention, it is possible to stably produce hydrocarbon with high efficiency from fluctuating renewable energy power by combining a hydrocarbonization reaction of CO₂ and water vapor electrolysis.

### Brief Description of Drawings

[FIG. 1] FIG. 1 is a block diagram of a CO₂ conversion system according to an embodiment of the present invention.
[FIG. 2] FIG. 2 shows an example of a cross-sectional structure of a heat storage unit.
[FIG. 3A] FIG. 3A shows another example of a cross-sectional structure of a heat storage unit.
[FIG. 3B] FIG. 3B shows still another example of the cross-sectional structure of the heat storage unit.
[FIG. 4A] FIG. 4A shows a perspective view of one form of the heat storage unit.
[FIG. 4B] FIG. 4B shows a perspective view of another form of the heat storage unit.
[FIG. 5] FIG. 5 is a block diagram according to another embodiment of a CO₂ conversion system.

### Description of Embodiments

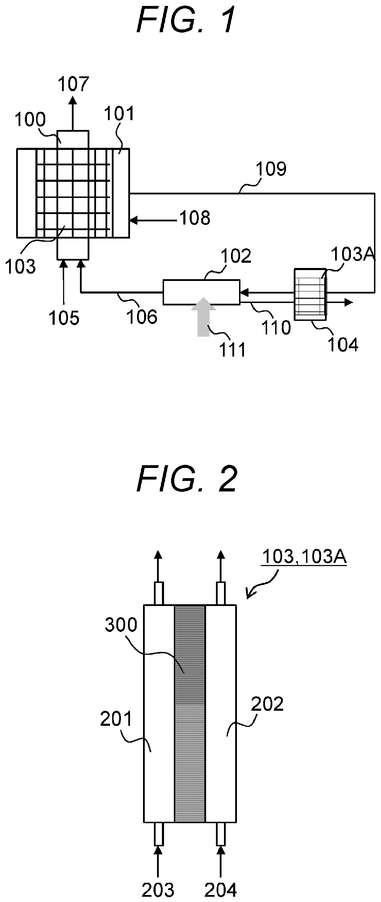

Hereinafter, embodiments of the present invention will be described. FIG. 1 is a block diagram of a CO₂ conversion system according to an embodiment of the present invention. This system includes SOEC (solid oxide electrolysis cell: water electrolysis device) 102 that electrolyzes water vapor 109 supplied from an evaporator 101 with a fluctuating electric power 111 derived from renewable energy to generate hydrogen (electrolytic hydrogen) 106, a tubular reactor (reaction tube) 100 that causes hydrogen 106 supplied from the SOEC 102 and CO₂ 105 to react with each other under a catalyst to convert CO₂ into hydrocarbon (fuel) 107, an evaporator 101 that vaporizes liquid water 108 by reaction heat generation in the reactor 100, and a heat exchanger 104 that heats the water vapor 109 from the evaporator 101 with exhaust gas 110 of the SOEC 102 and supplies the heated water vapor to the SOEC 102. An example of the CO₂ 105 may be, for example, exhaust gas from a coal-fired power plant.

The reactor 100 and the evaporator 101 include a heat storage unit 103 and are combined, for example, integrated with each other. The heat storage unit 103 includes a heat storage material described later. The heat storage unit 103A is also provided in the heat exchanger 104. The water vapor 109 generated in the evaporator 101 is input to the heat exchanger 104, is subjected to heat exchange with the high-temperature gas 110 from the SOEC 102 to have a higher temperature, and is supplied to the SOEC 102.

In the reactor 100, a catalyst for converting the CO₂ 105 into the hydrocarbon 107 by the hydrogen 106 is mounted. The structure of the hydrocarbon varies depending on the type of catalyst. For example, when Ni/Al₂O₃ is used as the catalyst, methane is generated from hydrogen and CO₂. CO may be used instead of CO₂, or both of them may be used. CO₂ and CO may be collectively referred to as a reaction gas for a hydrocarbonization reaction or a carbon source gas.

The reaction for producing hydrocarbon from hydrogen and CO₂ is an exothermic reaction, and the hydrocarbonization reaction is promoted as the temperature rises with the progress of the reaction. On the other hand, when the reaction reaches a high temperature range, the main reaction is the production of CO from hydrocarbon. Since the heat generation of the hydrocarbonization reaction is cooled by the heat of vaporization when the evaporator 101 converts water into water vapor, the reactor 100 is controlled to a temperature suitable for the hydrocarbonization reaction.

As an example, in the case of the methanation reaction, the methanation reaction is started from around 200°C, and CO is mainly generated from around 500°C. Therefore, the reactor 100 controls the hydrocarbonization reaction to 200 to 500°C. More preferably, the temperature is preferably controlled at 250 to 400°C at which a proportion of methane in a reaction product is more than half.

The SOEC 102 performs high temperature electrolysis with steam as a highly efficient process. In order for the SOEC 102 to perform electrolysis at a high temperature of 600 to 900°C, the heat exchanger 104 exchanges heat of high temperature exhaust (400 to 800°C) from the SOEC 102 with the water vapor 109 to maintain the water vapor at a high temperature and supply with the water vapor to the SOEC 102.

When the fluctuating electric power derived from renewable energy decreases, electrolysis in the SOEC 102 is suppressed, and an amount of hydrogen 106 decreases. In order to prevent the temperature of the reactor 100 from lowering due to the decrease in the amount of hydrogen and the hydrocarbonization reaction from stopping, it is necessary to limit the amount of water supplied to the evaporator 101 so as not to fall below a temperature range suitable for the hydrocarbonization reaction. When the fluctuating electric power increases beyond a prediction range, the supply amount of the water 108 may be increased.

Due to the limitation of the supply amount of water, when the amount of water vapor generated by the evaporator 101 decreases, the amount of water vapor to the SOEC 102 decreases, and together with the decrease in power, the electrolysis of the SOEC 102 is further suppressed. In addition, the amount of gas from the SOEC 102 to the heat exchanger 104 decreases, the amount of heat exchanged with water vapor also decreases, and the temperature rise of water vapor is insufficient. When a temperature of a steam electrolytic unit of the SOEC 102 rapidly decreases, the steam electrolytic unit may be damaged.

Thus, the heat storage unit 103 for the reactor 100 provides the amount of heat stored based on the reaction heat of the hydrocarbonization reaction to the environment of the hydrocarbonization reaction while the fluctuating electric power 111 decreases, and as a result, a temperature suitable for the hydrocarbonization reaction is maintained without limiting the supply amount of water.

In addition, the heat storage unit 103A of the heat exchanger 104 also provides the amount of heat stored based on the heat of the exhaust gas 110 from the SOEC 102 before the fluctuating electric power 111 decreases to the water vapor 109 while the fluctuating electric power decreases, thereby suppressing the temperature decrease of the water vapor 109.

The system of FIG. 1 provides a heat storage unit 103 including a heat storage material in at least one of the reactor 100 and the heat exchanger 104 as a region requiring temperature control. There are various types of heat storage methods of the heat storage material, and there are sensible heat storage, latent heat storage, and chemical heat storage when the heat storage methods are roughly classified. Sensible heat storage utilizes specific heat of a substance, latent heat storage utilizes transition heat (latent heat) associated with phase change and transition of a substance, and utilizes the transition heat (latent heat) associated with phase change and transition of a thermal substance, and chemical heat storage utilizes endotherm and exotherm during chemical reaction (absorption, mixing, hydration).

Latent heat storage has a higher heat storage density than sensible heat storage, can supply heat at a constant phase transition temperature, basically repeats a phase transition of a stable, safe, and inexpensive substance as compared with chemical heat storage, and thus is easy and excellent also in terms of durability. Since the temperature of the reactor 100 is controlled to 250 to 400°C and the temperature of the heat exchanger 104 is controlled to 400°C or higher (400°C to 600°C), a latent heat storage material is more preferable than a sensible heat storage material having a large temperature change, and chemical heat storage may be used. Although heat generated by the methanation reaction in the reactor 100 may be stored by sensible heat of a catalyst carrier in the reactor, it is difficult to compensate for the temperature decrease in the reactor 100 with this heat storage amount.

As the latent heat storage material, materials that can be selected are limited in a temperature range to be used. In particular, in a high temperature region of 250°C or higher, there are few options for organic substances, and therefore it is preferable to select an inorganic substance, particularly a salt compound or a metal material.

An inorganic substance that undergoes phase transition in the range of 250 to 400°C may be selected for the heat storage unit 103 for the reactor 100. For example, a nitrate salt compound such as KNO₃ having a melting point of 307°C or NaNO₃ having a melting point of 337°C may be used. Although one specific material may be used, a mixed material may be used because the melting point can be adjusted by mixing with another material. Examples of the material to be mixed include LiNO₃ and NaNO₂. In addition, a hydroxide such as NaOH having a melting point of 318°C may be used.

Although examples of the metal material include lead having a melting point of 327.5°C as a simple metal, lead is not preferable because of toxicity. Since the melting point of the metal can be adjusted when the metal forms an alloy with another metal, the metal is preferably used as an alloy. Examples of the metal material that can be utilized in a region requiring temperature control of the reactor 100 include alloys such as Zn, Al, Mg, Ag, Sn, and Cu. In a Mg-Zn-based alloy in which Mg having a melting point of 650°C and Zn having a melting point of 419°C are mixed at 49: 51 weight percent, the melting point is around 342°C, and therefore, the Mg-Zn-based alloy can be used as a heat storage material.

For the heat storage unit 103A for the heat exchanger 104, an inorganic substance that undergoes phase transition at a temperature equal to or higher than 400°C necessary for temperature control within a range of a gas temperature of 400 to 800°C input from the SOEC may be selected.

For example, a chloride such as MgCl₂ having a melting point of 714°C or KCl having a melting point of 770°C may be used. Although one specific material may be used, a mixed material may be used because a phase change temperature range can be adjusted by mixing with another material. Examples of the metal include Al having a melting point of 660°C and Mg having a melting point of 650°C. In addition, an alloy such as Al, Mg, Cu, or Si may be used.

As described above, the heat storage units 103 and 103A maintain the reactor 100 and the heat exchanger 104 at optimum temperatures by containing phase change materials having different melting points. The shape and form of the heat storage unit are not limited as long as heat can be transferred from a high-temperature fluid to a low-temperature fluid.

FIG. 2 shows an example of a cross-sectional structure of the heat storage units 103 and 103A. The heat storage region 300 occupies a space sandwiched between a high-temperature fluid circulation unit 201 through which the high-temperature fluid flows and a low-temperature fluid circulation unit 202 through which the low-temperature fluid flows, and an internal space of the heat storage region 300 is filled with the above-described heat storage material. An amount of the heat storage material filled may be appropriately set in accordance with a target value or a design value of a heat storage capacity.

The high-temperature fluid circulation unit 201 of the heat storage unit 103 circulates hydrocarbon 203 produced by reaction of hydrogen and carbon dioxide under a catalyst. The low-temperature fluid circulation unit 202 circulates water 204 (108 in FIG. 1), and converts the water into water vapor by heat from the high-temperature fluid via the heat storage region 300. In the heat exchanger 104, high-temperature gas 203 (110 in FIG. 1) from the SOEC 102 flows through the high-temperature fluid circulation unit 201, and the water vapor 204 (109 in FIG. 1) flows through the low-temperature fluid circulation unit 202.

An occupied volume of the heat storage material in the heat storage region 300 is limited to a range in which the heat storage region 300 and the fluid circulation units 201 and 202 on both sides adjacent to the heat storage region are not destroyed at the time of volume expansion of the heat storage material because a volume change due to a phase change between a solid and a liquid varies depending on the material.

The surface of the high-temperature and low-temperature fluid circulation units 201 and 202 may be subjected to a treatment for obtaining a high specific surface area in order to increase heat conduction efficiency, or a structure having a high specific surface area may be installed. In order to increase surface roughness, the surface may be mechanically roughened or chemically roughened by etching or the like. In addition, a structure such as a fin or a honeycomb may be installed. The heat storage material may be molded into a powder, a pellet, or the like.

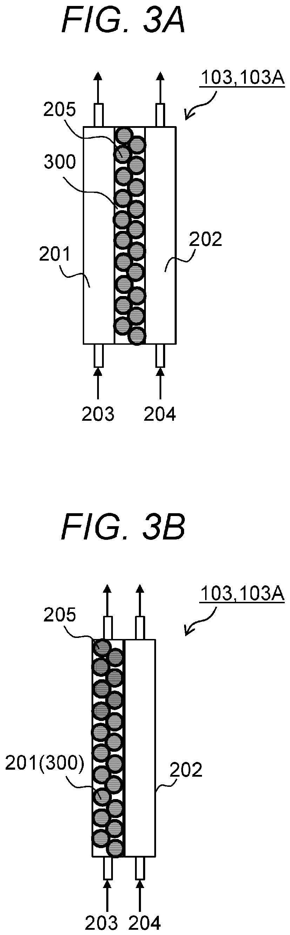

FIG. 3A shows another example of a cross-sectional structure of the heat storage units 103 and 103A. The heat storage material is contained in a ceramic or metal sphere 205 having a high melting point, and is filled in the internal space of the heat storage region 300. Although a heat capacity of the heat storage material decreases due to the presence of the sphere, heat conductivity is kept constant, and therefore stable heat conduction is expected even when a flow amount of the high-temperature fluid decreases.

It is also possible to integrate the heat storage region 300 and the fluid circulation unit by filling the sphere 205 in the fluid circulation unit. Since the fluid directly flows through the surfaces of the plurality of spheres, the conduction efficiency is improved. FIG. 3B shows a form in which the high-temperature fluid circulation unit 201 is filled with the sphere 205. The low-temperature fluid circulation unit 202 may be filled with the sphere. Both the circulation units may be filled with the sphere. In order to increase the thermal conduction efficiency of the surface of the fluid circulation unit not filled with the sphere, a measure for improving the specific surface area of the surface, for example, an increase in surface roughness, a chemical etching treatment of the surface, addition of a fin or a honeycomb, or the like may be applied.

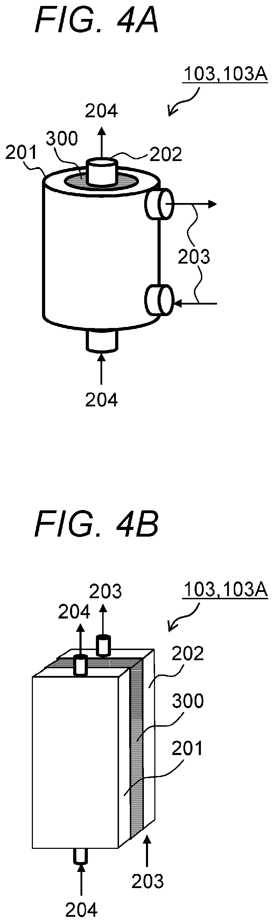

FIG. 4A shows a perspective view of one form of the heat storage units 103 and 103A, and FIG. 4B shows a perspective view of another form of the heat storage units 103 and 103A. As shown in FIG. 4A, the heat storage region 300 and the fluid circulation units 201 and 202 may have a cylindrical shape or a rectangular shape as shown in FIG. 4B. In FIG. 4A, the heat storage region 300 has a hollow cylindrical shape, the low-temperature fluid circulation unit 202 is inserted into the inner periphery, and the high-temperature fluid circulation unit 201 having a cylindrical shape exists on the outer periphery of the heat storage region 300. An outer peripheral portion of the high-temperature fluid circulation unit 201 may be covered with a heat insulating material so that heat does not leak to the outside. Note that the high-temperature fluid circulation unit 201 may be disposed on the inner peripheral side of the heat storage region 300, and the low-temperature fluid circulation unit 202 may be disposed on the outer peripheral side.

In FIG. 4B, the heat storage region 300 and the fluid circulation units 201 and 202 each have a rectangular shape, and the heat storage region 300 is sandwiched between the high-temperature fluid circulation unit 201 and the low-temperature fluid circulation unit 202. According to the form of the heat storage unit shown in FIGS. 4A and 4B, since the entire two opposing surfaces (an inner peripheral surface and an outer peripheral surface in FIG. 4A, and a front surface and a back surface in FIG. 4B) of the heat storage region 300 are in contact with the high-temperature fluid circulation unit or the low-temperature fluid circulation unit, the heat conduction between the fluid circulation unit and the heat storage region is efficiently performed.

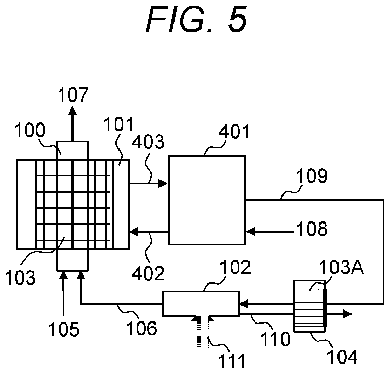

FIG. 5 shows another form of the system of FIG. 1. The system of FIG. 5 differs from the system of FIG. 1 in that the system of FIG. 5 includes, instead of the evaporator (101) of FIG. 1, a cooler 101 that supplies a low-temperature oil (refrigerant) 402 to the reactor 100 and cools a reaction field of the hydrocarbonization reaction to deliver the high-temperature oil 403, and a heat exchanger 401 that performs heat exchange between the high-temperature oil 403 and the water 108 to deliver the water vapor 109. As described above, even if the temperature of the reactor 100 is controlled by a circulation type refrigerant, the same effect as that in FIG. 1 can be achieved.

Hereinafter, the present invention will be described more specifically based on Examples and Comparative Examples.

### <Example 1>

As the heat storage material, KNO₃ was used for the reactor 100, and an Al-Si-Cu alloy having a melting point of around 520°C was used for the heat exchanger 104. The heat storage material was formed into a pellet, and filled in the heat storage units 103 and 103A. The heat storage units 103 and 103A had a cylindrical structure (FIG. 4A).

The SOEC 102 can produce H₂ with a maximum flow rate of 60 L/min by input of 15 kW of power from renewable energy, and the reactor 100 can produce methane with a flow rate of 15 L/min by H₂ and CO₂ with a flow rate of 15 L/min. At this time, about 110 kJ/min of heat is generated from the reactor 100. This amount of heat allows the evaporator 101 to convert 90 g/min of water into water vapor. The heat storage material (KNO₃: 6.5 kg) filled in the heat storage unit 103 can maintain the temperature of the reactor 100 in a range suitable for the methanation reaction for a predetermined time.

When 60 L/min of H₂ and 15 L/min of CO₂ were heated to 250°C and input to the reactor 100, synthesis of methane was confirmed at an outlet of the reactor 100. The temperature of the reactor 100 when a methane production amount reached the value described above was about 300°C, and it was confirmed that the temperature of the methanation reaction could be controlled. When liquid water was sprayed and introduced into the evaporator 101, generation of vapor was confirmed.

The SOEC 102 was operated at 750°C, and oxygen gas generated from the SOEC 102 was input to the heat exchanger 104. In the heat exchanger 104, the temperature could be controlled at approximately 520°C. When the water vapor 109 was input to the heat exchanger 104, the temperature of the heat exchanger 104 was raised to about 600°C.

In this state, when the input power was reduced to 1/10, that is, 1.5 kW, the generation of H₂ was reduced to 6 L/min. When CO₂ was also set to 1/10 in accordance with the amount of H₂ generated and input to the reactor, the methane generation amount was reduced. The temperature of the reactor 100 could be maintained for 10 minutes by the heat storage unit. In addition, when the input amount of water was also set to 1/10 in accordance with the fluctuation of the input power, it was confirmed that the temperature of the reactor 100 could be maintained for about 100 minutes. From this, it was confirmed that water vapor could be generated for 1 hour or more necessary for suppressing breakage of the steam electrolytic unit of the SOEC 102.

As described above, in the system described above, even if the input power fluctuates due to the fluctuation of the renewable energy, a high-temperature water vapor can be supplied to the SOEC 102 while continuing the CO₂ conversion during the same period.

### <Example 2>

As the heat storage material, a ceramic sphere containing a Mg-Al alloy was used for the reactor 100, and a ceramic sphere containing an Al-Si alloy having a melting point of around 580°C was used for the heat exchanger 104. The structure of the heat storage unit was a plate shape (FIG. 4B). The same results as in Example 1 could be obtained.

### <Comparative Example 1>

A system was constructed in the same manner as in Example 1 except that the heat storage units 103 and 103A were not filled with the heat storage material. When a mixed gas of H₂ and CO₂ in a volume ratio of 4: 1 was prepared, heated to 250°C, and introduced into the reactor 100, production of methane was confirmed at the outlet of the reactor 100. As the methanation reaction proceeded, the temperature of the reactor increased, and liquid water was sprayed to the evaporator 101 exceeding 550°C, so that generation of vapor was confirmed. Next, when the electric power 111 was reduced to 1/10, the temperature of the reactor 100 rapidly decreased to stop the methanation reaction. Since the generation of water vapor was also stopped, there was no input of the water vapor 109 to the SOEC 102, and the SOEC 102 was also stopped.

### <Comparative Example 2>

Conditions and states were similar to those of Comparative Example 1 except that the system of FIG. 5 was used. When the input power was increased by 1/10 as in Comparative Example 1, the cooling effect by the oil was increased; therefore, as in Comparative Example 1, the temperature of the reactor 100 rapidly decreased, and the methanation reaction was stopped.

The descriptions of the embodiments show specific examples of the contents of the present invention, the present invention is not limited to these descriptions, and various changes and modifications by those skilled in the art can be made within the scope of the technical idea disclosed in the present specification.

### Reference Signs List

- 100: reactor (reaction tube)
- 101: evaporator
- 102: SOEC
- 103: heat storage unit
- 104: heat exchanger
- 105: CO₂
- 106: H₂
- 107: hydrocarbon
- 108: liquid water
- 109: water vapor
- 110: gas generated from SOEC

## Claims

1. A system for converting CO₂ into fuel, the system comprising:
a water electrolysis apparatus that generates hydrogen by electrolyzing water vapor using a fluctuating electric power derived from renewable energy;
a reactor that reacts CO₂ with the hydrogen to generate hydrocarbon;
an evaporator that evaporates water, by heat generated by the reaction in the reactor, to generate water vapor;
a heat exchanger that heats the water vapor with exhaust gas from the water electrolysis apparatus and supplies the water vapor to the water electrolysis apparatus; and
a heat storage unit for the reactor, the heat storage unit including a heat storage material that stores heat generated by the reaction.

2. The system according to claim 1, further comprising a heat storage unit for the heat exchanger including a thermal storage material that stores heat of the heat exchanger.

3. The system according to claim 2, wherein a heat storage unit for the reactor and the heat storage unit for the heat exchanger each include the material that stores heat by phase change or chemical change.

4. The system according to claim 3, wherein a phase change temperature region of the heat storage material of the heat storage unit for the reactor is lower than a phase change temperature region of the heat storage material of the heat storage unit for the heat exchanger.

5. The system according to claim 1, wherein the heat storage unit for the reactor is capable of maintaining a temperature of the reactor in a temperature range suitable for the reaction when the fluctuating electric power decreases.

6. The system according to claim 1, wherein the heat storage unit for the heat exchanger is capable of maintaining a temperature of the heat exchanger in a temperature range suitable for an electrolytic reaction for generating hydrogen from water vapor when the fluctuating electric power decreases.

7. The system according to claim 4, wherein a melting point of the thermal storage material of the heat storage unit for the reactor is in a range of 200 to 500°C.

8. The system according to claim 4, wherein a melting point of the thermal storage material of the heat storage unit for the heat exchanger is in a range of 400 to 800°C.

9. The system according to claim 1, wherein
the thermal storage material of the heat storage unit for the reactor includes at least one of:
a nitrate or hydroxide salt; or
an alloy including any one or more of Zn, Al, Mg, Ag, Sn, and Cu.

10. The system according to claim 2, wherein
the thermal storage material of the heat storage unit for the heat exchanger includes at least one of:
a nitrate or hydroxide salt; or
an alloy including any one or more of Al, Mg, Cu, and Si.

11. A method for converting CO₂ into fuel, the method comprising:
an electrolysis step of electrolyzing water vapor using a fluctuating electric power derived from renewable energy to generate hydrogen;
a hydrocarbonization step of reacting CO₂ with the hydrogen to generate hydrocarbon;
a water vapor generation step of evaporating water, by heat generated by the reaction for generating the hydrocarbon, to generate water vapor;
a water vapor heating step of heating the water vapor generated in the water vapor generation step with a high-temperature gas generated in the electrolysis step and then supplying the water vapor to the electrolysis step; and
a heat storage step of storing the heat generated in the hydrocarbonization step in a heat storage material and radiating heat from the heat storage material to continue the hydrocarbonization step and the water vapor generation step when the fluctuating electric power decreases.
